(19) Europäisches Patentamt
European Patent Office
Office européen des brevets

(11) **EP 4 382 039 A1**

(12) **EUROPEAN PATENT APPLICATION**

(43) Date of publication:
**12.06.2024   Bulletin 2024/24**

(21) Application number: **22211623.8**

(22) Date of filing: **06.12.2022**

(51) International Patent Classification (IPC):
**A61B 5/024** (2006.01)     **A61B 5/00** (2006.01)

(52) Cooperative Patent Classification (CPC):
**A61B 5/02416; A61B 5/0205; A61B 5/029;
A61B 5/08; A61B 5/14551; A61B 5/7257;
A61B 5/7278**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventor: **VAN KESTEREN, Hans Willem
Eindhoven (NL)**

(74) Representative: **Philips Intellectual Property &
Standards
High Tech Campus 52
5656 AG Eindhoven (NL)**

(54)   **METHOD FOR DERIVING A MEASURE OF PPG VARIABILITY**

(57)     A method for deriving a measure of PPG variability which is indicative of fluid responsiveness. The PPG signal is processed with an adaptive re-sampling algorithm which resamples the signal with a sampling density which is a function of instantaneous pulse rate. When the PPG signal is subsequently transformed with a discrete frequency-domain transform such as an FFT, the resulting frequency spectrum reflects a variability of the sample in which the FM contribution is largely eliminated. This provides variability information which is more reflective of the AM contribution which is the dominant contribution associated with respiration.

FIG. 4

**Description**

FIELD OF THE INVENTION

This invention relates to a method for deriving a measure of PPG variability.

BACKGROUND OF THE INVENTION

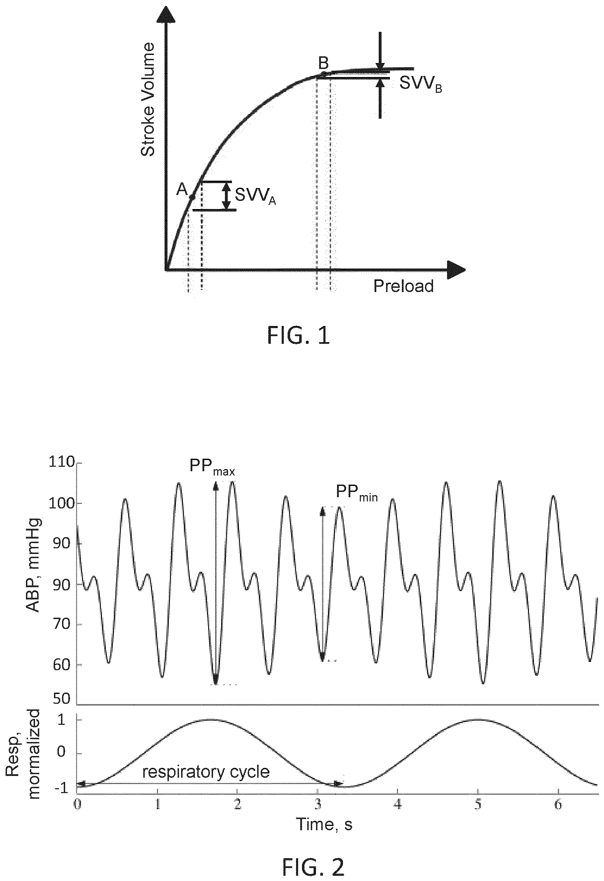

The maintenance of adequate oxygen delivery across the body is a core part of critical care medicine. Fluid administration is often employed in hemodynamic management to improve Cardiac Output (CO). The more that the heart muscle is stretched during filling, the greater the volume of blood pumped into the aorta. The fluid responsiveness of a patient depends upon the ability of the heart to adapt to increasing volumes. This is illustrated in Fig. 1 which shows the so-called Frank-Starling curve which relates stroke volume (y-axis) with cardiac pre-load (x-axis).

If the heart is operating at the steep portion of this curve (e.g. point A indicated in Fig. 1, low preload), an increase in preload (by volume expansion) will result in a significant increase in Stroke Volume (SV). If the heart is operating at the plateau region of the curve (e.g. point B, higher preload) the fluid responsiveness will be low.

Fluid responsiveness can be assessed using an SV or CO measurement before and after fluid administration. This requires the injection of a fluid bolus with a risk for volume overload.

Cardiopulmonary interactions have been shown to be useful in predicting fluid responsiveness. Differences in intrathoracic pressure during the respiration cycle result in changes in SV upon respiration. The SV Variation (SVV) is larger at point A ($SVV_A$) of the curve of Fig. 1 than point B ($SVV_B$). SV and Arterial Blood Pressure (ABP) exhibit a strong correlation and a respiration modulation of the Pulse Pressure (PP) is also present.

Photo Plethysmography (PPG) can also be used to assess this variability. Fig. 2 illustrates the relationship between ABP and respiration cycle. Fig. 2 shows modulation of arterial blood pressure during the respiration cycle and shows that a maximum PP value, $PP_{MAX}$, coincides with a maximum respiration and a minimum PP value, $PP_{MIN}$, coincides with a minimum respiration. Pulse pressure is the amplitude of a pulse wave.

For mechanically ventilated patients, fluid responsiveness can be predicted using a measurement of SVV, pulse pressure variation (PPV) and/or variability of a PPG signal ('PPG variability') upon respiration. By way of example, PPG variability indices include Pulse Oximetry Plethysmography (POP) variability and/or the Masimo's proprietary index PVI (Pleth Variability Index). Threshold values for fluid responders and non-responders have been obtained for both of these indices in several clinical studies. For spontaneously breathing patients this variability method is less effective since the respiration modulation effect is less pronounced and varies from breath to breath during respiration. Pre-load redistributing maneuvers such as a passive leg raise test are also used in clinical practice to predict fluid responsiveness but considered to have less operative performance.

In the current state of the art, SVV provides the strongest predictive indicator for fluid responsiveness, followed by PPV, followed by PPG variability. However, an SVV measurement requires an invasive central line, and so is generally only practicable for the patients in the most critical condition. For PPV measurements, a radial artery line is sufficient, but is still invasive. Non-invasive pulse pressure measurements are also possible, but generally have reduced signal quality. PPG variability as a proxy signal for fluid responsiveness has the advantage of fully non-invasive measurement and also measurement through a pulse oximeter which is widely available and inexpensive. However, at present, PPG variability provides the least accurate predictive signal of fluid responsiveness.

It would be of benefit to provide an improved measure of PPG variability so that it can be used as a more reliably predictive measure of fluid responsiveness.

SUMMARY OF THE INVENTION

The invention is defined by the claims.

According to examples in accordance with an aspect of the invention, there is provided a method for deriving a plethysmography variability measure, the method comprising:

obtaining a plethysmography signal of a subject, comprising a sampled time series of plethysmography sensor measurements spanning a time window, and the time window spanning a plurality of pulse cycles of the subject;

obtaining an instantaneous pulse rate signal for the patient spanning said time window;

applying a re-sampling algorithm to the sampled time-series of the plethysmography signal, wherein the resampling algorithm is adapted to perform sample rate modification based, at least in part, on the instantaneous pulse rate across the time window, wherein the resampling algorithm re-samples the plethysmography signal across the time window at a re-sampling rate which is a function of said instantaneous pulse rate;

applying a discrete time-to-frequency-domain transform to the re-sampled plethysmography signal waveform to obtain a frequency spectrum;

identifying a pre-determined set of frequency component peaks in the frequency spectrum;

calculating a plethysmography variability measure based on a frequency amplitude of said identified peaks in the frequency spectrum.

Optionally the method may further comprise generating a data output based on the calculated plethysmography variability measure for export.

With regards to the plethysmography signal, discussions in this document may refer more particularly to a Photoplethysmography (PPG) signal. However, the inventive principle is applicable more broadly to any type of plethysmography signal and plethysmography sensor including for example a Speckle plethysmography (SPG) sensor. Thus, any reference to a PPG sensor, PPG signal or PPG variability measure should be read as encompassing use with any plethysmography sensor or signal.

For reasons that will become clear in the description to follow, the re-sampling operation has the effect of substantially eliminating a frequency modulation (FM) contribution to the plethysmography variability within the discrete frequency transform. Only amplitude modulation (AM) is closely correlated with fluid responsiveness, and thus eliminating FM substantially improves accuracy and reliability of the resulting plethysmography variability measure as an indicator for fluid responsiveness.

Embodiments of the present invention differ from known algorithms in employing adaptive resampling and deriving the variability measure through frequency domain analysis. This deviates from state-of-the art variability algorithms which use time-domain and peak/trough identification.

A discrete frequency domain transform converts a finite sequence of equally-spaced samples of a function into a same-length sequence of equally-spaced samples of an output frequency spectrum. Thus, the discrete transform assumes equally spaced input samples. By modifying the sampling density of the input function (the PPG signal) in a way that is correlated with the FM contribution, the FM contribution can be eliminated in the frequency domain transform.

The discrete time-to-frequency-domain transform may in some embodiments be a discrete Fourier transform, e.g. a Fast Fourier Transform (FFT).

The plethysmography variability in the present context may refer primarily to the variability upon respiration or ventilation, i.e. respiratory plethysmography variability, or respiration-associated plethysmography variability.

The resampling algorithm is adapted to perform sample rate modification of the plethysmography signal across the time window to derive a re-sampled plethysmography signal, and wherein the sample density of the re-sampled signal across the time window is a function of the instantaneous pulse rate over the time window.

The re-sampling algorithm is thus an adaptive resampling algorithm. An adaptive re-sampling means a re-sampling in which the re-sampling rate or density is adjustable or variable, or may vary according to a defined function.

The time window may be a moving time window. This allows for example a real-time plethysmography variability measure to be obtained and output from the method, i.e. a plethysmography variability measure as a function of time.

Although a PPG sensor is referred to above, in other examples, an SPG sensor could be used. The effects of the invention remain unchanged.

In some embodiments, the method may additionally comprise performing a filtering operation in advance of the re-sampling.

Here, the obtaining a plethysmography signal may comprise: obtaining a red sensor signal waveform and an infrared sensor signal waveform from the plethysmography sensor. The method may comprise applying an adaptive digital filter to the red sensor signal waveform and infrared sensor signal waveform from the plethysmography sensor. An adaptive digital filter means a digital filter with coefficients which are dynamically adjustable or variable.

In some embodiments, the coefficients of the adaptive digital filter may be configured based on a measure correlated with an SpO2 value for the patient. In some examples, this measure might be the ratio

$$r_{SpO2} = \frac{AC \text{ of } PPG_R}{DC \text{ of } PPG_R} \div \frac{AC \text{ of } PPG_{IR}}{DC \text{ of } PPG_{IR}}$$

. This will be explained further later.

In some embodiments, the plethysmography signal may be a plethysmography signal obtained from a plethysmography sensor is disposed at a central body location of the patient, for example the forehead of the patient. A measurement at a central site like provides a high-quality signal and avoids presents of waveform changes which arise when blood travels from the heart to a peripheral measurement site.

In some embodiments, the instantaneous pulse rate signal may be computed from beat-to-beat time intervals in the plethysmography signal.

Optionally, in some embodiments, the obtaining the instantaneous pulse rate signal may further comprises use of a respiration signal for the subject. Respiration cycle is correlated with pulse rate and thus can be used to enhance reliability or resolution of the instantaneous pulse rate signal.

The instantaneous pulse rate signal is provided as an input to the resampling algorithm and is used to define the resampling rate or density across the time window.

The above-described adaptive-resampling algorithm re-samples the plethysmography signal at an increased rate when the instantaneous pulse rate is higher and at a decreased rate when the instantaneous pulse rate is lower.

The resampling algorithm may for example comprise defining a set of (re)sampling points with a sampling density proportional to instantaneous pulse rate and subsequently defining the plethysmography signal values for each sampling point based on interpolation from the originally measured plethysmography sample points.

In some embodiments, the resampling operation comprises: defining a number of re-sample points for the time window; distributing the re-sample points across the time window with a density in dependence upon an instantaneous pulse rate as a function of time across the time window; and determining a plethysmography signal value for each re-sample point based on interpolation from the sampled time series of measurements of the originally obtained plethysmography signal.

In some embodiments, the number of re-sample points may be set as equal to the number sample points in the original plethysmography signal, so that the average sampling rate over the time window remains unchanged.

Alternatively, the number of re-sample points might be varied. In some embodiments, the number of re-sample points can be made a function of average respiration rate across the time window.

One way of doing this is to make the number of sample points across a time window spanning each respiration cycle proportional to the period of the respiration cycle.

For example, in some embodiments, the re-sampling algorithm comprises: receiving a respiration signal for the subject, the respiration signal representing a parameter correlated with a respiration cycle phase; identifying a time window in the plethysmography signal which spans a single respiration cycle; defining a number of re-sample points for the time window in proportion to a period of said single respiration cycle; distributing the re-sample points across the time window in dependence upon an instantaneous pulse rate as a function of time across the time window; and determining a plethysmography signal value for each re-sample point based on interpolation from the sampled time series of measurements of the originally obtained plethysmography signal.

In some embodiments, the earlier mentioned pre-determined set of frequency component peaks may correspond to: frequency component $(PPG(f)|_{PR})$ corresponding to a pulse rate of the subject, frequency component $(PPG(f)|_{PR+RR})$ corresponding to a sum of the pulse rate and a respiration rate, and frequency component $(PGG(f)|_{PR-RR})$ corresponding to the pulse rate minus the respiration rate.

In some embodiments, the plethysmography variability measure (PPGV) may be calculated based on evaluating the equation:

$$PPGV = \frac{\{PPG(f)|_{PR+RR} + PPG(f)|_{PR-RR}\}}{PPG(f)|_{PR}}$$

where $(PPG(f)|_{PR})$ is the amplitude of the frequency component corresponding to a pulse rate of the subject, $(PPG(f)|_{PR+RR})$ is the amplitude of the frequency component corresponding to a sum of the pulse rate and a respiration rate, and $(PGG(f)|_{PR-RR})$ is the amplitude of the frequency component corresponding to the pulse rate minus the respiration rate.

In some embodiments, the identifying the peaks may simply comprise identifying all peaks in the frequency spectrum exceeding a threshold amplitude, on the assumption that the frequency spectrum for the re-sampling plethysmography signal only contains peaks at these frequency points. In simple cases this assumption is indeed correct.

However, in some cases, it may be more difficult to identify the relevant peaks or there may be additional peaks due to a source of systematic noise in the signal. This is especially relevant for spontaneously breathing patients with variations in breathing volume and respiration rate.

To improve peak identification, in some embodiments, the method comprises obtaining a respiration signal for the subject, the respiration signal representing a parameter correlated with a respiration cycle phase. The set of frequency component peaks may be identified in or selected from the frequency spectrum based on applying a peak identification algorithm which uses the respiration signal as an input. The respiration signal can be used to compute respiration rate, RR. The pulse rate, PR, is already obtained as part of the method, as discussed above.

In some embodiments, the obtaining the respiration signal (for any purpose described in this document) may comprise: receiving a temperature signal from a temperature sensor placed adjacent an airway of the subject and mapping oscillations in the temperature sensor signal to oscillations in a respiration cycle of the subject to derive the respiration signal.

In some embodiments, the temperature sensor may be integrated in a common sensor unit with the plethysmography sensor.

Another aspect of the invention is a computer program product comprising computer program code configured, when executed by a processor, to cause the processor to perform a method in accordance with any embodiment described in this document or in accordance with any claim of this application.

Another aspect of the invention is a processing device, comprising: an input/output; and one or more processors operatively coupled to the input/output.

The one or more processors are adapted to: obtain a plethysmography signal of a subject, comprising a sampled time series of plethysmography sensor measurements spanning a time window, and the time window spanning a plurality of pulse cycles of the subject; obtain an instantaneous pulse rate signal for the patient spanning said time window; apply a re-sampling algorithm to the sampled time-series of the plethysmography signal, wherein the resampling algorithm is adapted to perform sample rate modification based, at least in part, on the instantaneous pulse rate across the time window, wherein the resampling algorithm re-samples the plethysmography signal across the time window at a re-sampling rate which is a function of said instantaneous pulse rate;

apply a discrete time-to-frequency-domain transform to the re-sampled plethysmography signal waveform to obtain a frequency spectrum; identify a pre-determined set of frequency component peaks in the frequency spectrum; calculate a plethysmography variability measure based on a frequency amplitude of said identified peaks in the frequency spectrum; and preferably generate a data output based on the calculated plethysmography variability measure.

Another aspect of the invention is a system comprising: the processing device outlined above or in accordance with any embodiment in this document; and a plethysmography sensor, for example a PPG sensor.

In some embodiments, the system may further comprise a sensor for measuring a parameter correlated with a respiration cycle phase, i.e. a respiration signal. In some embodiments, the sensor comprises a temperature sensor for placement adjacent an airway of the subject, and optionally wherein the sensor comprises a sensing unit which comprises both the plethysmography sensor and temperature sensor.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

   Fig. 1 shows a curve which relates stroke volume with cardiac pre-load;
   Fig. 2 shows an example PPG signal as a function of time over two respiration cycles;
   Fig. 3 illustrates different types of signal modulation which may be present in a PPG signal;
   Fig. 4 shows a flow diagram of an example method according to one or more embodiments of the invention;
   Fig. 5 is a block diagram of an example processing device and system according to one or more embodiments of the invention;
   Fig. 6 shows a flow diagram of an example method according to one or more embodiments;
   Fig. 7 illustrates a re-sampling operation in which sampling density is varied as a function of instantaneous pulse rate;
   Fig. 8 illustrates identification of frequency peaks in the frequency spectrum of the re-sampled PPG signal;
   Fig. 9 illustrates an example optional filtering operation;
   Fig. 10 shows a processing flow of an example method according to one or more embodiments;
   Fig. 11 shows a processing flow of an example method according to a further one or more embodiments;

and
   Fig. 12 shows a processing flow of an example method according to a further one or more embodiments.

DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for deriving a measure of plethysmography variability which is better indicative of fluid responsiveness. This is achieved by pre-processing the plethysmography signal with an adaptive re-sampling algorithm which re-samples the signal with a sampling density which is a function of instantaneous pulse rate. When the plethysmography signal is subsequently transformed with a discrete frequency-domain transform such as an FFT, the resulting frequency spectrum reflects a variability of the sample in which the FM contribution is largely eliminated. This provides variability information which is more reflective of the AM contribution which is the dominant contribution associated with respiration. This in turn provides variability information more closely indicative of fluid responsiveness.

Examples and embodiments discussed herein refer particularly to a PPG signal and PPG sensor. However, embodiments of the invention can employ use of any type of plethysmography sensor, including for example Speckle plethysmography sensor (SPG). Thus it should be understood that any reference below to a PPG signal, PPG sensor, or PPG variability measure can be replaced with reference more broadly to plethysmography signal, plethysmography sensor or plethysmography variability measure without loss of technical effect.

Embodiments of the invention provide a plethysmography variability measure which is a more accurate a reliable indicator of fluid responsiveness. The proposed variability measure is effective for all classes of patient. Variability measurements in the state of the art have been limited to patients on mechanical ventilation. To improve outcome in mechanical ventilation, the use of low tidal volumes has become more common in clinical practice. Embodiments of the present invention allow for measurements at low-tidal volumes, where signal-to-noise for variability signals has in the past been low and thus quality limited. Variability measurements at low tidal volumes also enable embodiments of the invention to be applied to spontaneously breathing patients. Spontaneously breathing patients also show variation in tidal volume and respiration rate. Embodiments of the invention provide an effective variability measure in cases where breathing volume and respiration rate may be varying.

As discussed above, a plethysmography signal exhibits modulations in its waveform correlated with progression of the respiratory cycle. These are referred to as respiratory modulations. Both the instantaneous frequency and amplitude of the plethysmography signal modulate as a function of respiratory cycle progression.

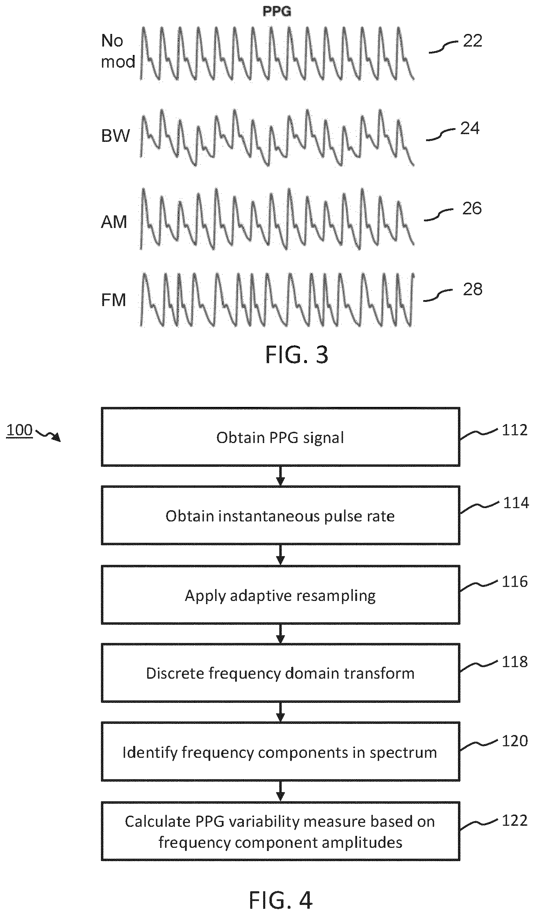

The respiratory modulations of an example photoplethysmography (PPG) signal are illustrated in Fig. 3. Three types of modulations in the signal can be discriminated: Baseline Wander (BW), Amplitude Modulation (AM), and Frequency Modulation (FM). Signal trace 22 in Fig. 3 shows a PPG signal with no modulation. Signal trace 24 shows a PPG signal with baseline wander (BW) modulation only. Baseline wander means modulation in the baseline (i.e. the DC offset) of the signal. Signal trace 26 shows a PPG signal with amplitude modulation (AM) only. Signal trace 28 shows a PPG signal with frequency modulation (FM) only. A real PPG signal could typically be expected to exhibit a mix of all these types of modulation. Fig. 3 shows them separated for purposes of illustration.

BW in a PPG signal is mainly reflective of changes in blood volume caused by respiratory changes in arterial blood pressure as well as respiratory induced vasoconstriction transferring blood to the veins. Baseline oscillations at frequencies below the respiration frequency can also be present due to sympathetic nervous system activity. PPG sensor movement can also impact the tissue blood volumes in the detection region and result in irregular fluctuations in the PPG baseline.

The FM of the PPG signal is also known as respiratory sinus arrhythmia. Several mechanisms contribute to the FM. These include for instance a baroreflex mediated change in heart rate upon stroke volume changes. Other contributing factors can include heart rate increases during stretching of the sinoatrial node.

The AM of the PPG signal is primarily reflective of changes in preload upon respiration. Thus, following from discussions above, it will be immediately recognized that the AM of the PPG signal is the most relevant type of modulation for measuring fluid responsiveness. Ideally, a PPG variation index (PPGV) which is to be used as a measure for fluid responsiveness would be based as far as possible on the Amplitude Modulation (AM) of the underlying PPG signal.

Embodiments of the present invention propose a method for processing the PPG signal to derive a PPG variability index which is based on selectively suppressing other types of modulation, and yielding a variability signal which is mostly indicative of PPG AM modulation.

A method and algorithm applied to calculate such a PPG variability measure should ideally be able to discriminate AM from other types of modulation and also ideally to discriminate variations caused by artefacts, e.g. motion artefacts. Additionally, the variations caused by venous pulsations are ideally also suppressed from the PPGV index.

A further important factor to consider is the delay between the airway pressure and consequent effects on different types of modulation. Airway pressure has an impact on the arterial blood pressure, and this manifests as a BW component after a certain time delay. Airway pressure also has an impact on the AM after a certain time delay, and thus occurs through application of pressure on the vena cava inflow at the right ventricle, and this in turn affecting the outflow and aortic pulse pressure at the left ventricle. The delay between the airway pressure and the AM effect in the PPG signal may be referred to as the pulmonary transit time.

State-of-the-art methods and algorithms to calculate PPV and PPG variability are based on identifying peaks in the time domain.

A different method to calculate PPG variability is described in the following paper: Acosta et al, Biomedical Signal Processing and Control 60 (2020) 101947. This describes a frequency-domain algorithm which recovers PPV values at signal-to-noise ratios six time smaller than time-domain algorithms. The algorithm is based on convolved components of cardiac and respiratory oscillations. Although, the method takes into account the pulmonary transit time and enables PPV measurement at low tidal volumes, the method has the limitation that it handles BW and AM in the PPG signal only. It is not able to accurately determine PPV when both AM and FM are present because these modulations mix in a complicated way in the Fourier Transform (e.g. FFT) spectrum for AM and FM values that occur in clinical practice.

Fig. 4 outlines in block diagram form steps of an example computer-implemented method 100 according to one or more embodiments. The steps will be recited in summary, before being explained further in the form of example embodiments.

The method 100 is for deriving a measure of plethysmogram variability. Variability in the context of the present invention may refer to variability upon respiration or ventilation. Ultimately, the aim to is to derive a plethysmogram variability measure which is reflective of (correlated with) fluid responsiveness.

The method comprises obtaining 112 a plethysmogram or plethysmography signal of a subject comprising a sampled time series of plethysmogram sensor measurements spanning a time window, and the time window spanning a plurality of pulse cycles of the subject. This may be a PPG signal in some embodiments.

The method further comprises obtaining 114 an instantaneous pulse rate signal for the patient spanning said time window. As will be explained later, this can be obtained from the plethysmogram signal itself, optionally in combination with a separate measurement source, such as a respiration sensor.

The method further comprises applying 166 a re-sampling algorithm to the sampled time-series of the plethysmogram signal. The resampling algorithm is adapted to perform sample rate modification, wherein the sample rate modification is based, at least in part, on the instantaneous pulse rate. The resampling algorithm re-samples the plethysmogram signal at a re-sampling rate which is a function of said instantaneous pulse rate. In other words, the plethysmogram signal is resampled across the time window and wherein the sampling density across the time window is a function of the instantaneous pulse rate across the time window.

The method further comprises applying 118 a discrete time-to-frequency-domain transform (e.g. a Fourier transform) to the re-sampled plethysmogram signal waveform to obtain a frequency spectrum. The effect of the aforementioned re-sampling is to substantially eliminate the FM modulation in the discrete frequency-domain transformed signal. A discrete frequency-domain transform assumes equally spaced sample points. Thus, applying a discrete transform to the plethysmogram signal with sampling density which varies as a function of pulse rate effectively eliminates the frequency modulation in the signal.

The method further comprises identifying 120 a pre-determined set of frequency component peaks in the frequency spectrum.

The method further comprises calculating 122 a plethysmogram variability measure based on an amplitude of said identified peaks in the frequency spectrum.

The method may further comprise generating a data output based on the calculated PPG variability measure. For example, the data output might be communicated to a user interface or datastore or external server.

With regards to the resampling, this is an adaptive resampling, where the resampling algorithm is adapted to perform sample rate modification of the plethysmogram signal across the time window to derive a re-sampled plethysmogram signal, and wherein the sample rate modification is a function of the instantaneous pulse rate over the time window.

The adaptive-resampling algorithm re-samples the plethysmogram signal at an increased rate when the instantaneous pulse rate is higher and at a decreased rate when the instantaneous pulse rate is lower.

The time-series may span a moving time window so that a real-time plethysmogram variability index is derived as a function of time.

Although a photoplethysmography (PPG) signal is referred to in examples presented herein, embodiments of the invention would work equally well with any other type of plethysmography signal, such as a speckle plethysmography (SPG) signal.

As noted above, the method can also be embodied in hardware form, for example in the form of a processing device which is configured to carry out a method in accordance with any example or embodiment described in this document, or in accordance with any claim of this application.

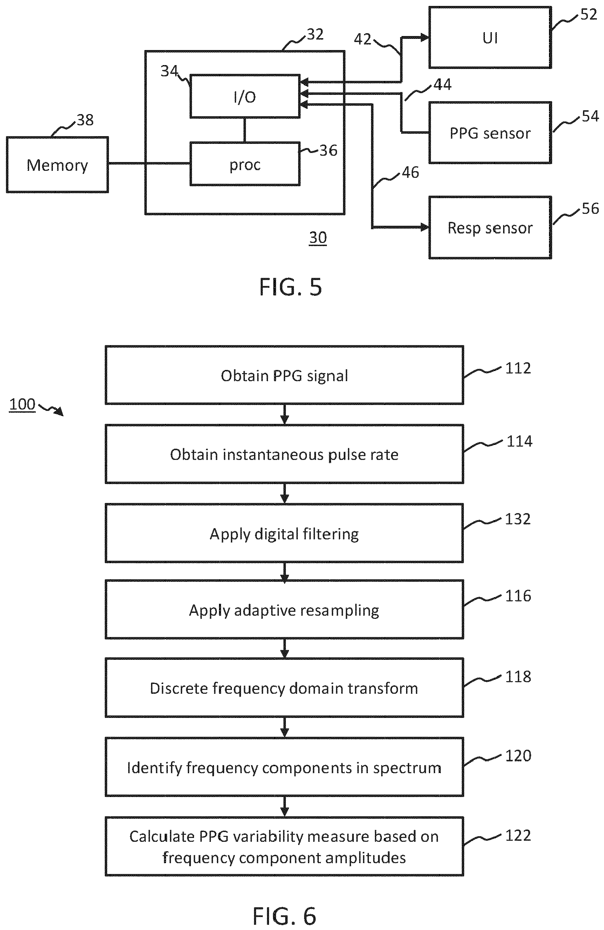

To further aid understanding, Fig. 5 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system does not have to comprise all of the illustrated hardware components; it may just comprise a subset of them.

The processing device comprises one or more processors 36 configured to perform a method in accordance with that outlined above, or in accordance with any embodiment described in this document or any claim of this application. In the illustrated example, the processing device further comprises a communication interface or an input/output 34.

As illustrated in the example of Fig. 5, the system 30 may further comprises a user interface 52 which includes a display unit. This may be used to display the computed plethysmogram variability measure.

The system may further comprise a plethysmogram sensor for generating the plethysmogram signal. This may be a PPG sensor in some embodiments. An SPG sensor is a further option. The plethysmogram sensor may be adapted for mounting or attachment at a central body location of the patient, for example the forehead of the patient or a nose of the patient.

The system 30 may further comprise a sensor 56 for generating a respiration signal indicative of respiration. For example a respiration signal may be representative of respiration cycle phase. In some examples this is a temperature sensor for placement adjacent an airway of the subject and wherein the processing device is adapted to map oscillations in the temperature sensor signal to oscillations in a respiration cycle of the subject to derive the respiration signal. In some embodiments, the respiration sensor 56 may be integrated in a same sensor unit as the plethysmography sensor 54.

Of course, it is not essential that the system 30 comprise all of these hardware components. A system in accordance with the invention can be provided which comprises none or only one or more of these components.

The input/output 34 is adapted for receiving the previously mentioned plethysmogram signal. It might alternatively be received from a datastore, or from an intermediary communication device, such as a hub server or a network node.

The system 30 may further comprise a memory 38 for storing computer program code (i.e. computer-executable code) which is configured for causing the one or more processors 36 of the processing device 32 to perform the method as outlined above, or in accordance with any embodiment described in this disclosure, or in accordance with any claim.

As mentioned previously, the invention can also be embodied in software form. Thus another aspect of the invention is a computer program product comprising code means configured, when run on a processor, to cause the processor to perform a method in accordance with any example or embodiment of the invention described in this document, or in accordance with any claim of this patent application.

In accordance with a preferred set of embodiments the method 100 further comprises applying a filtering operation to the obtained plethysmogram signal in advance of the resampling 116.

Fig. 6 outlines steps of a further example method in accordance with one or more embodiments where such a filtering operation 132 is included as an additional step. The method is otherwise the same as that outlined in Fig. 4.

In more detail, according to some embodiments, the filtering operation 132 may be implemented in the following way. Here, reference will be made specifically to a photoplethysmogram (PPG) sensor for brevity, but other types of plethysmogram signal can be used.

The aforementioned obtaining 112 of a PPG signal may comprise obtaining a red sensor signal waveform and an infrared sensor signal waveform from the PPG sensor. The PPG signal is thus composed of two components. Then the method may further comprise applying an adaptive digital filter to the red sensor signal waveform and an infrared sensor signal waveform from the PPG sensor. The filtered PPG signal (i.e. the PPG signal with filtered red and IR components) is then used for the remainder of the method.

With regards to the coefficients for the adaptive digital filter, preferably the coefficients of the adaptive digital filter are configured based on a measure correlated with or indicative of an SpO2 value for the patient. This will be explained in more detail to follow.

As discussed above, the method involves a re-sampling operation in which the plethysmography signal is resampled with a re-sampling rate which varies as a function of instantaneous pulse rate over the sampling time window. This is now discussed in more detail. Reference will be made in particular to a photoplethysmogram (PPG) signal, but the method is applicable more broadly.

The resampling comprises obtaining a signal indicative of instantaneous pulse rate, *pulse(t),* as a function of time over the course of a measurement time window. This can either be computed based on peak-to-peak separation in the PPG signal itself or based on an external measurement source.

The resampling operation then comprises processing the PPG signal over said measurement window to resample the signal with a re-sampling rate which varies across the time window as a function of the instantaneous pulse rate across the time window. Another way of expressing this is that the resampling operation comprises determining a resampling density function, s(t), which is a function of time over the time window, wherein the resampling density function is a function of the instantaneous pulse rate, i.e. correlated with the instantaneous pulse rate. In other words, resampling density is higher when the instantaneous pulse rate is higher and lower when the instantaneous pulse rate is lower. Once the resampling density function is determined, new (re)sample points are defined across the time window in accordance with the resampling density. Then, the PPG signal value for each new resample point is set based on interpolation from the true PPG signal values of the originally obtained PPG signal. In this way, the frequency modulation in the PPG signal is compensated if the resampled PPG signal values were to be plotted against equidistant sample points. The corollary of this is that, in the discrete Fourier transform of the resampled signal (which assumes equally spaced sample points), the FM is substantially eliminated.

Thus, to summarize, in at least some embodiments, the resampling operation 116 comprises: defining a number of re-sample points for the time window; distributing the re-sample points across the time window with a sample density in dependence upon an instantaneous pulse rate as a function of time across the time window; and determining a PPG signal value for each re-sample point based on interpolation from the sampled time series of measurements of the originally obtained PPG signal.

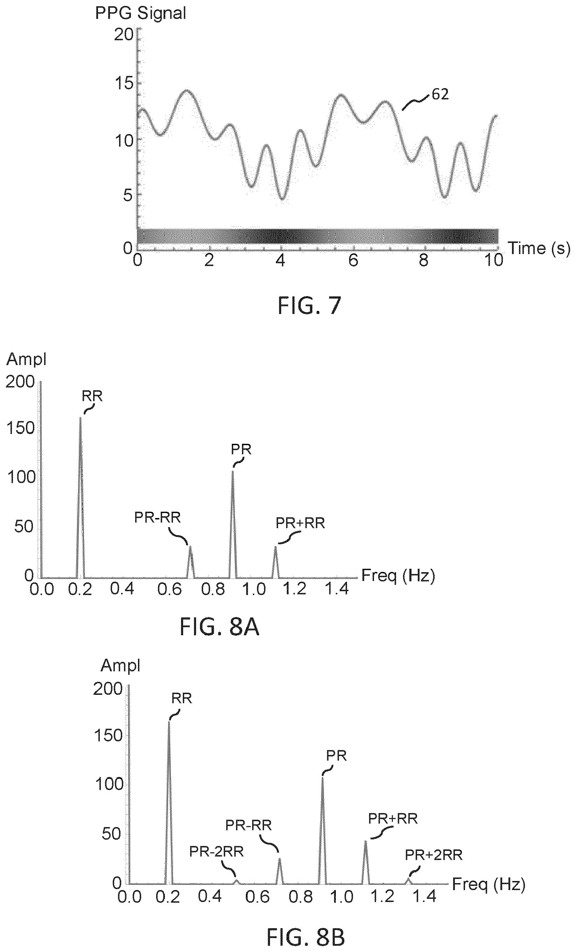

The re-sampling is illustrated by Fig. 7, where the grey levels indicate re-sampling density or re-sampling rate as a function of time over a time window. Darker values are associated with higher re-sampling rate and lighter values are associated with lower re-sampling rate. The waveform 62 is the example PPG signal. In this example, the instantaneous pulse rate can be inferred from the beat-to-beat interval, with a smaller interval indicating a higher pulse rate. It can be seen how for sections of the signal with lower instantaneous pulse rate (larger beat-to-beat interval), the resampling rate (or re-sampling density) is higher (darker grey values).

Thus, the proposed resampling algorithm resamples the PPG signal at an increased rate (increased sampling density) when the instantaneous pulse rate is higher and at a decreased rate when the pulse rate is lower. The average sampling rate over the time window may remain at a pre-set value, for example the same as in the original PPG signal. Thus, for example, the sampling rate might be made lower than the original signal in some areas and higher than the original signal in others. The time interval between subsequent (re)sample points is chosen to be proportional to the instantaneous beat-to-beat interval. The PPG signal strength for each new (re)sample point is obtained from an interpolation of the original samples. In this way the FM is compensated if the resampled PPG signal were (hypothetically) to be plotted against equidistant sample points and is absent in the discrete Fourier transform of the resampled PPG signal (which assumes equally spaced samples).

As discussed above, the method involves identifying a set of pre-defined frequency components in a frequency spectrum of the re-sampled PPG signal.

To obtain the frequency spectrum, a Fast Fourier Transform (FFT) (or other discrete frequency domain transform) is applied to convert the re-sampled PPG signal to the frequency domain.

A discrete transform assumes equally spaced sample points. Thus, the effect of a discrete transform on the re-sampled data series with samples whose spacing varies as a function of pulse rate, is to effectively eliminate the PPG frequency modulation (FM) in the discrete Fourier transform. This is because the FM is primarily caused by the variation in pulse rate. This is beneficial because, as discussed above, only the amplitude modulation component of PPG variability is connected to fluid responsiveness. Thus, where the target is a PPG variability index which is most closely reflective of fluid responsiveness, eliminating the FM component of PPG variability is desired.

With regards to the pre-defined frequency components to be identified, these can be understood as followed.

In the absence of frequency modulation (FM) in a PPG signal, the frequency spectrum for the PPG signal has peaks at frequencies corresponding to the respiration rate (RR), pulse rate (PR), at the sum of pulse rate and respiration rate (PR+RR) and at the difference between the pulse rate and the respiration rate (PR-RR).

These peaks in the frequency spectrum are indicated in Fig. 8A.

From these, a PPG variability measure (PPGV) which is reflective primarily of amplitude modulation, and thus reflective of fluid responsiveness, can be computed form the equation:

$$PPGV = \frac{\{PPG(f)|_{PR+RR} + PPG(f)|_{PR-RR}\}}{PPG(f)|_{PR}}$$

where $(PPG(f)|_{PR})$ is the amplitude of the frequency component corresponding to a pulse rate (PR) of the subject, $(PPG(f)|_{PR+RR})$ is the amplitude of the frequency component corresponding to a sum of the pulse rate and a respiration rate (PR+RR), and

$(PGG(f)|_{PR-RR})$ is the amplitude of the frequency component corresponding to the pulse rate minus the respiration rate (PR-RR).

This equation therefore effectively gives a value for the ratio of the sum of the PR±RR frequency amplitude peaks to the PR frequency amplitude peak.

Changes in pulmonary transit time influence the phases of the frequency domain peaks. However, the amplitudes of the frequency domain peaks remain insensitive to the pulmonary transit time. The presence of a small residual FM in the PPG signal is tolerable and does not have a major impact on accuracy. This is because this contributes to an equal and opposite change of the PR-RR and PR+RR peaks, with the result that the average is still unchanged and still reflects the reflects the AM.

By contrast, at larger FM ratios, the AM, transpulmonary delay and FM mix in a complex way in the frequency spectrum and the average of PR-RR and PR+RR can no longer be used to extract a PPG variability index reflecting the AM.

Furthermore, if the AM contribution to PPG variation has not been properly removed, additional peaks appear in the frequency spectrum of the PPG signal. In particular, peaks are observable also at PR±n.RR with n≥2. This is illustrated in Fig. 8B. The adaptive resampling described above removes the FM contribution and thus eliminates these additional peaks. Thus, if the re-sampling has successfully removed the FM modulation in the Fourier-transformed PPG signal, then the additional peaks at PR±n.RR with n≥2 should be absent.

In some embodiments, the method may comprise detecting whether the frequency spectrum contains frequency component peaks at frequencies of PR±n.RR with n≥2. This can be used as a check for successful elimination of the FM modulation in the PPG signal. If the additional peaks are detected, the re-sampling operation could be re-run again, or additional filtering could be performed, or the relevant signal portion (i.e. for the relevant current time window) might be discarded and the method repeated for the next time window. The absence of peaks at PR±n.RR with n≥2 can thus be used as feedback on the appropriate suppression of FM by the adaptive resampling algorithm.

In some embodiments, to implement the step of selecting or identifying the relevant peaks in the frequency spectrum, additional use may be made of a respiration signal for the subject, from which a respiration rate for the subject can be determined. Use might also be made of the pulse rate signal which is already acquired as an earlier part of the method. The set of frequency component peaks can then be selected from the frequency spectrum based on applying a peak identification algorithm which uses the respiration signal and pulse rate signal as an input. The respiration signal may be obtained from a respiration sensing means, which could be an airflow sensing means positioned at an airway, such as a temperature sensor positioned at an airway. Alternatively, the PPG sensor signal itself can be used to derive a respiration rate through methods which are well known.

Alternatively, the method may take an approach wherein it is assumed that the frequency spectrum for the resampled signal contains peaks only at the four frequencies RR, PR, PR-RR and PR+RR and thus simply identifies the amplitude of the complete set of peaks which are present in the spectrum. Which peak corresponds to which of the four physiological markers can be determined by the order of the peaks: i.e. the first peak is RR (typically the lowest frequency), second peak is PR-RR, third peak is PR, fourth peak is PR+RR.

As mentioned previously, in some embodiments, a filtering operation is additionally applied to the obtained PPG signal in advance of the resampling operation. Details for an example implementation of such a filtering option will now be discussed.

Preferably the filtering operation employs use of an adaptive digital filter, wherein the coefficients of the digital filter are configured in dependence upon an input to the filter. The coefficients may be configured based on a measure correlated with or indicative of an SpO2 value for the patient.

The aforementioned obtaining 112 of a PPG signal may comprise obtaining a red sensor signal waveform and an infrared sensor signal waveform from the PPG sensor.

The method may further comprise applying an adaptive digital filter to the red sensor signal waveform and the infrared sensor signal waveform from the PPG sensor. The filtered PPG signal (i.e. the PPG signal with filtered red and IR components) is then used for the remainder of the method.

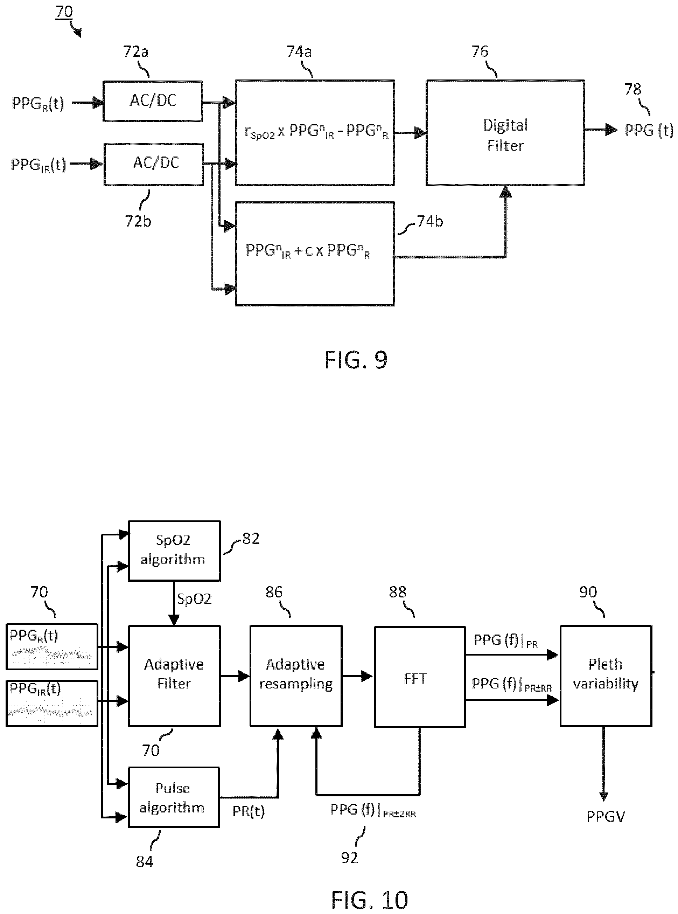

Fig. 9 shows a block diagram of the processing steps of an example adaptive filter operation 70 for cleaning the red $PPG_R(t)$ and infrared $PPG_{IR}(t)$ signals of the PPG sensor signal.

An input is received indicative of a measure which is related or correlated with an $SpO_2$ value for the subject. This can be computed from the unfiltered PPG signal using known techniques. One example is described further below.

With reference to Fig. 9, the AC components of the red, $PPG_R(t)$, and infrared, $PPG_{IR}(t)$, signals are normalized by their DC components. In other words, the AC component is extracted from each signal and divided by the DC component. This is indicated by processing steps 72a and 72b in Fig. 9.

Following this, difference 74a and sum 74b signals of the two normalized signals are calculated.

The difference signal 74a is computed as:

$$dif^n = r_{SpO2} \cdot PPG^n{}_{IR} - PPG^n{}_R$$

where $r_{SpO2} = \dfrac{AC\ of\ PPG_R}{DC\ of\ PPG_R} \div \dfrac{AC\ of\ PPG_{IR}}{DC\ of\ PPG_{IR}}$ and $PPG^n{}_{IR}$ is the PPG signal sample $n$ of the $PPG_{IR}$ signal and $PPG^n{}_R$ is the PPG signal sample $n$ of the $PPG_R$ signal.

The ratio $r_{SpO2}$ is a common measure derived in pulse oximetry, and is correlated with or indicative of SpO2. The actual SpO2 value is normally calculated from the $r_{SpO2}$ value using a sensor type specific calibration equation. However, for the purposes of the filter the ratio $r_{SpO2}$ is sufficient.

The sum signal 74b is computed as

$$sum^n = PPG^n{}_{IR} + c \cdot PPG^n{}_R$$

where c is a constant which can be tuned as desired.

The difference spectrum calculated from 74a in this way reflects the non-arterial and noise components and is used to determine the digital filter coefficients. The sum signal 74b is obtained by adding a fraction c of the red signal to the infrared signal. This fraction can be zero, but preferably c is set to obtain the highest signal-to-noise ratio for the sum signal. The cleaned PPG(t) is obtained by passing the sum signal through the digital filter 76, using the difference signal to define the coefficients.

The result of the filtering operation is to leave a time domain PPG signal having the non-arterial components removed.

An example implementation of the method according to at least one set of embodiments will now be illustrated, with reference to Fig. 10. It will be recognized that not all features of this particular implementation are essential to the general inventive concept, and the various details are presented by way of illustration of at least one example implementation. In particular, as already mentioned, the filtering operation 70 is optional and so could be omitted from the embodiment of Fig. 10 if preferred.

Fig. 10 shows a block diagram outlining steps of an example method for computing a PPG variability measure which is indicative of fluid responsiveness.

As indicated as 70, the method comprises receiving as input a PPG signal for a subject comprising a red PPG signal, $PPG_R(t)$, and an infrared PPG signal, $PPG_{IR}(t)$.

The method may further comprise a processing operation for filtering the red and infrared signals. This leaves a time domain PPG signal having the non-arterial components removed. This may for example be done with an adaptive filter 70, for example a filter as described above with reference to Fig. 9. The example of Fig. 10 includes an SpO2 algorithm 82 for computing a measure correlated with or indicative of an SpO2 value for the patient. This is computed in this example from the input red and infrared PPG signals. For example, in the adaptive filter of Fig. 9, the measure correlated with or indicative of SpO2 was the ratio

$r_{SpO2} = \dfrac{AC\ of\ PPG_R}{DC\ of\ PPG_R} \div \dfrac{AC\ of\ PPG_{IR}}{DC\ of\ PPG_{IR}}$. Thus, the SpO2 algorithm 82 may output a computed value of the ratio $r_{SpO2}$ in some embodiments.

The $SpO_2$ algorithm 82 may compute the SpO2 measure for example with a running time window, e.g. several seconds. This is common for computation of $SpO_2$.

If the filter 70 is omitted the SpO2 algorithm 82 may also be omitted.

In parallel to the $SpO_2$ algorithm 82, a pulse algorithm 84 may additionally be run which is configured to compute an instantaneous pulse rate. This is also computed using the red and infrared PPG signals based on determining beat-to-beat interval within the red and/or infrared PPG signals. The pulse algorithm may operate with a running time window, e.g. of several seconds. This time-domain algorithm 84 determines the beat-to-beat intervals (instantaneous pulse rate). As already discussed, the instantaneous pulse rate is used as an input to the adaptive re-sampling operation (providing information about the FM modulation depth of the PPG signal 70).

Due to noise in the PPG signal, the instantaneous beat-to-beat interval can sometimes be inaccurate and therefore optionally a smoothing operation can additionally be applied to overcome this issue.

Following the optional filtering operation 70, the filtered signals are supplied as input to an adaptive re-sampling algorithm 86 which is adapted to re-sample the signal with a re-sampling rate which is a function of the instantaneous pulse rate. Thus, the output of the aforementioned pulse algorithm 84 is provided as an input to the adaptive re-sampling algorithm 86. As discussed above, the adaptive re-sampling algorithm removes or at least significantly reduces the FM contribution to the PPG signal variability. As discussed above, this is an aim of the overall method because only primarily the AM contribution to the PPG variability is reflective of fluid responsiveness. The details of the adaptive resampling operation have already been described above, and thus will not be repeated here.

The re-sampled PPG signal is processed by a discrete time-to-frequency-domain transform 88, such as a Fast Fourier Transform (FFT). This yields a frequency spectrum for the re-sampled PPG signal. As discussed above, since the FFT assumes equally spaced samples, the Fourier transform of the signal re-sampled based on pulse rate yields a frequency spectrum with the FM modulation substantially removed.

From the frequency spectrum, a pre-defined set of peaks are extracted and used to compute 90 a PPG variability measure. In particular, peaks at least at frequencies corresponding to pulse rate (PR) and Pulse Rate (PR) +/- Respiration rate (RR) may be used. For example, a PPG variability measure, PPGV, might be computed as:

$$PPGV = \frac{\{PPG(f)|_{PR+RR} + PPG(f)|_{PR-RR}\}}{PPG(f)|_{PR}}$$

where $(PPG(f)|_{PR})$ is the amplitude of the frequency component corresponding to a pulse rate (PR) of the subject, $(PPG(f)|_{PR+RR})$ is the amplitude of the frequency component corresponding to a sum of the pulse rate and a respiration rate (PR+RR), and

$(PGG(f)|_{PR-RR})$ is the amplitude of the frequency component corresponding to the pulse rate minus the respiration rate (PR-RR). The details for this have already been described above.

As also mentioned above, in some embodiments, the presence in the frequency spectrum of additional peaks at PR +/- 2RR might be used as a check of successful FM elimination from the PPG signal, and wherein the adaptive resampling might be repeated if peaks at PR +/- 2RR exceeding a certain threshold amplitude are detected. This is indicated schematically by feedback path 92. This feature is optional.

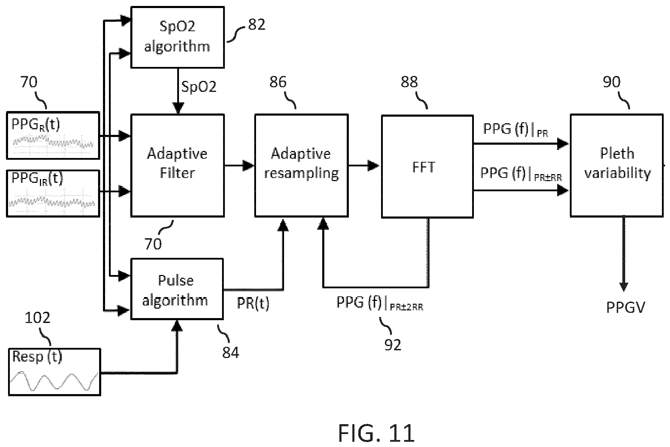

The processing flow according to a further example method according to one or more embodiments is schematically shown in Fig. 11.

This method is the same as that of Fig. 10 described above, except that an additional input is provided of a respiration signal 102 for the subject obtained from a separate respiration measurement source. Again, not all elements are essential. In particular, at least the filtering operation 70, the SpO2 algorithm 82 and the feedback path 92 are not essential and could be omitted.

The separate respiration signal can serve any of a number of different purposes.

In some embodiments the separate respiration signal 102 can be used to improve accuracy of the peak selection procedure discussed above. This has already been discussed above.

In some embodiments, and as indicated in Fig. 11, the respiration signal 102 can be used to improve reliability of the instantaneous pulse rate computation 84.

In particular, deriving the instantaneous pulse rate may comprise: determining beat-to-beat intervals from the PPG signal 70, correlating the beat-to-beat intervals with the respiratory signal, and deriving a final instantaneous pulse rate measure based on the combination of the respiratory signal and the PPG signal. In particular, it can be difficult to compute a real-time instantaneous pulse rate that can take account of pulse rate variation using only PPG beat-to-beat intervals. This is because the sample intervals are relatively large compared to the scale on which pulse rate variation might take place. However, it is also known that real-time short-scale variation in a person's pulse rate is correlated with the respiratory cycle. Thus, by using the respiration signal, variation in pulse rate on a shorter scale might be better detectable. One approach for example is to compute a first series of instantaneous pulse rate sample points using the PPG beat-to-beat interval times over a measurement window. This set of sample points can then be further supplemented with additional sample points computed from the respiration signal, by assuming that pulse rate will vary in correlation with respiration cycle. In other words, an initial instantaneous pulse rate signal computed from the PPG signal alone can be modulated in correlation with modulation in the subject's respiration cycle. Consequently, a high-quality instantaneous pulse rate reference signal can be provided to the adaptive re-sampling algorithm 86.

With regards to the sources for the respiration signal 102, for mechanically ventilated patients, the volume or pressure signal from the ventilator can be used.

For spontaneously breathing patients, the respiration measurement could for example be derived from a temperature sensor (e.g. a thermistor) positioned at an airway of the patient, e.g. a nasal airway of the patient. In some embodiments, the temperature sensor may be incorporated on an nasal alar $SpO_2$ sensor. The temperature sensor reacts to temperature differences in the respiratory air during inhalation and exhalation. A capnography sensor is a further option for providing a respiration input.

Thus, in summary, according to one or more embodiments, the obtaining the instantaneous pulse rate signal may comprise: obtaining a respiration signal for the subject and processing the respiration signal in combination with the PPG signal to derive the instantaneous pulse rate signal. In some embodiments, the obtaining the respiration signal may comprise receiving a temperature signal from a temperature sensor placed adjacent an airway of the subject and mapping oscillations in the temperature sensor signal to oscillations in a respiration cycle of the subject to derive the respiration signal.

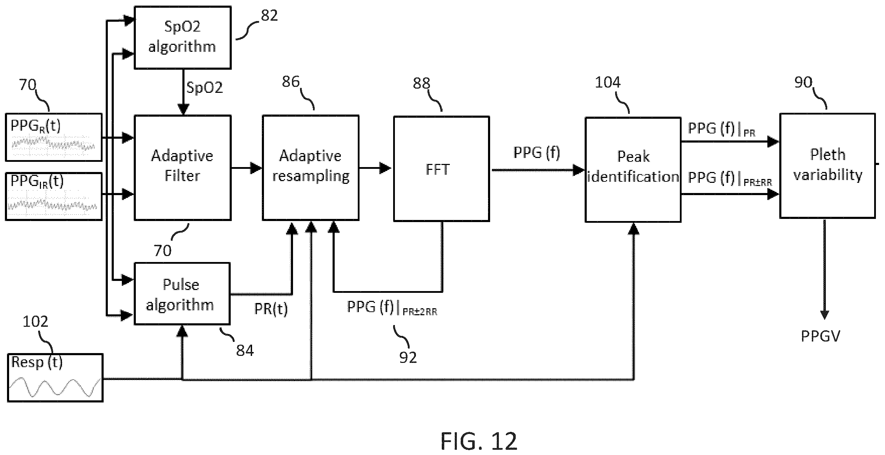

The processing flow according to a further example method according to one or more embodiments is schematically shown in Fig. 12.

This method may be the same as that of Fig. 10 or Fig. 11 described above, except that the resampling operation 86 and peak identification operation might be supplemented in the manner described below. Again, not all elements of the shown method flow are essential. For example, at least the filtering operation 70, the SpO2 algorithm 82, the feedback path 92 and the use of the respiration signal 102 in the calculation of the instantaneous pulse rate signal are not essential and could be omitted.

For spontaneously breathing patients, the respiration-cycle period varies from breath to breath in an irregular way. This results in a more distributed intensity across the frequency spectrum than for ventilated patients and makes the derived PPGV value less accurate. To overcome this issue, a two-stage adaptive resampling can be implemented.

A respiration signal 102 is provided as input to the adaptive resampling algorithm. To implement the two-stage resampling, in a first step a total number of (re)sampling points for the PPG signal over a time window spanning a single respiration cycle is determined as a function of the period of the respiration cycle. In other words, the number of PPG sample points over a respiration cycle is made proportional to the period of the respiration cycle. In a second step, the sample points during each given respiration cycle are distributed over the respiration cycle according to the instantaneous pulse rate, i.e. the sample density is made a function of the instantaneous pulse rate over the respiration cycle.

Subsequently, the PPG signal intensity is determined for each of the distributed (re)sample time points based on interpolation from the original PPG samples.

In cases where the average pulse rate and average respiration rate are close in frequency, it becomes more difficult to identify the RR, PR and PR±RR peaks in the frequency spectrum. To account for this, a peak identification algorithm 104 can be added configured to determine the spectral amplitudes of these peaks. This algorithm receives as input a respiration signal 102, and computes an average respiration rate over two or more respiration cycles. From this, the RR, PR and PR±RR frequencies can be explicitly calculated and the amplitude values corresponding to these frequencies read off to provide the inputs to computing the PPGV measure in the way described above.

The two-stage adaptive resampling described above and the additional peak identification procedure described above are functionally tied to one another and thus example embodiments could include one or both or neither of these optional features.

Although examples described above have referred to use of a photoplethysmography (PPG) signal, in fact any plethysmography signal could be used. For example, the method could be applied equally well to a Speckle Plethysmography (SPG) signal. The latter is similar to PPG technology, except that a coherent laser source is used instead of an LED and an image sensor is used for speckle (interference) detection instead of a single photodetector. Studies have shown that SPG provides a higher signal-to-noise than PPG and a more pressure-like waveform is obtained. Both could provide an advantage in obtaining a higher quality variability index for fluid responsiveness.

If an SPG signal is used, the optionally adaptive filter 70 and SpO2 algorithm 82 described above can be omitted. All other features are fully compatible with use of an SPG signal.

In combination with any of the above described embodiments, further improvements in reliability of the PPG variability measure could be obtained by normalizing the variability measure by a measure of the breathing volume of the subject. This has the effect of compensating for patient-specific breathing volumes and also differences in breathing volume during ventilation compared with spontaneous breathing. If a Fourier transform is used in the method, this normalization can be implemented by division of the PPG variability measure by the amplitude of the respiration rate (RR) peak in the frequency spectrum of the resampled PPG signal.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (100) for deriving a plethysmography variability measure, the method comprising:

    obtaining (112) a plethysmography signal for a subject, comprising a sampled time series of plethysmography sensor measurements spanning a time window, and the time window spanning a plurality of pulse cycles of the subject;
    obtaining (114) an instantaneous pulse rate signal for the subject spanning said time window;
    applying (116) a re-sampling algorithm to the sampled time-series of the plethysmography signal, wherein the resampling algorithm is adapted to perform sample rate modification based, at least in part, on the instantaneous pulse rate across the time window, wherein the resampling algorithm re-samples the plethysmography signal across the time window at a re-sampling rate which is a function of said instantaneous pulse rate;
    applying (118) a discrete time-to-frequency-domain transform to the re-sampled plethysmography signal waveform to obtain a frequency spectrum;
    identifying (120) a pre-determined set of frequency component peaks in the frequency spectrum;
    calculating (122) a plethysmography variability measure based on a frequency amplitude of said identified peaks in the frequency spectrum; and
    generating a data output based on the calculated plethysmography variability measure.

2. The method of claim 1 wherein the obtaining a plethysmography signal comprises:

    obtaining a red sensor signal waveform and an infrared sensor signal waveform from the plethysmography sensor; and
    applying a digital filter to the red sensor signal waveform and infrared sensor signal waveform from the plethysmography sensor;
    preferably wherein coefficients of the digital filter are configured based on a measure correlated with an SpO2 value for the patient.

3. The method of claim 2 wherein the plethysmography signal is obtained from a plethysmography sensor disposed at a central body location of the patient, for example the forehead of the patient.

4. The method of any of claims 1-3, wherein the obtaining the instantaneous pulse rate signal comprises detecting beat-to-beat time intervals in the plethysmography signal.

5. The method of any of claims 1-4, wherein the resampling algorithm comprises:

defining a number of re-sample points for the time window;

distributing the re-sample points across the time window in dependence upon an instantaneous pulse rate as a function of time across the time window;

determining a plethysmography signal value for each re-sample point based on interpolation from the sampled time series of measurements of the obtained plethysmography signal.

6. The method of any of claims 1-5, wherein the re-sampling algorithm comprises:

receiving a respiration signal for the subject, the respiration signal representing a parameter correlated with a respiration cycle phase;

identifying a time window in the plethysmography signal which spans a single respiration cycle;

defining a number of re-sample points for the time window in proportion to a period of said single respiration cycle;

distributing the re-sample points across the time window in dependence upon an instantaneous pulse rate as a function of time across the time window;

determining a plethysmography signal value for each re-sample point based on interpolation from the sampled time series of measurements of the obtained plethysmography signal.

7. The method of any of claims 1-6, wherein the pre-determined set of frequency component peaks correspond to:

a frequency component $(PPG(f)|_{PR})$ corresponding to a pulse rate of the subject,

a frequency component $(PPG(f)|_{PR+RR})$ corresponding to a sum of the pulse rate and a respiration rate, and

a frequency component $(PGG(f)|_{PR-RR})$ corresponding to the pulse rate minus the respiration rate.

8. The method of claim 7, wherein the plethysmography variability measure (PPGV) is calculated based on evaluating the equation:

$$PPGV = \frac{\{PPG(f)|_{PR+RR} + PPG(f)|_{PR-RR}\}}{PPG(f)|_{PR}}$$

9. The method of claim 7 or 8,

wherein the method comprises obtaining a respiration signal for the subject, the respiration signal representing a parameter correlated with a respiration cycle phase; and

wherein the set of frequency component peaks are selected from the frequency spectrum based on applying a peak identification algorithm which uses the respiration signal as an input.

10. The method of claim 9, wherein the obtaining the respiration signal comprises: receiving a temperature signal from a temperature sensor placed adjacent an airway of the subject and mapping oscillations in the temperature sensor signal to oscillations in a respiration cycle of the subject to derive the respiration signal.

11. A computer program product comprising computer program code configured, when executed by a processor, to cause the processor to perform a method in accordance with any of claims 1-10.

12. A processing device (32), comprising:

an input/output (34);
one or more processors (36) operatively coupled to the input/output, and adapted to:

obtain a plethysmography signal of a subject, comprising a sampled time series of plethysmography sensor measurements spanning a time window, and the time window spanning a plurality of pulse cycles of the subject;

obtain an instantaneous pulse rate signal for the patient spanning said time window;

apply a re-sampling algorithm to the sampled time-series of the plethysmography signal, wherein the resampling algorithm is adapted to perform sample rate modification based, at least in part, on the instantaneous pulse rate across the time window, wherein the resampling algorithm re-samples the plethysmography signal across the time window at a re-sampling rate which is a function of said instantaneous pulse rate;

apply a discrete time-to-frequency-domain transform to the re-sampled plethysmography signal waveform to obtain a frequency spectrum;

identify a pre-determined set of frequency component peaks in the frequency spectrum;

calculate a plethysmography variability measure based on a frequency amplitude of said identified peaks in the frequency spectrum; and

generate a data output based on the calculated plethysmography variability measure.

13. A system (30) comprising:

the processing device (32) of claim 12; and
a plethysmography sensor (54), for example a
PPG sensor.

14. The system (30) of claim 13, further comprising a sensor (56) for measuring a parameter correlated with a respiration cycle phase.

15. The system (30) of claim 14, wherein the sensor comprises a temperature sensor for placement adjacent an airway of the subject, and optionally wherein the sensor comprises a sensing unit which comprises both the plethysmography sensor and temperature sensor.

FIG. 1

FIG. 2

PPG

No mod

BW

AM

FM

22

24

26

28

FIG. 3

100

| Obtain PPG signal | 112 |

| Obtain instantaneous pulse rate | 114 |

| Apply adaptive resampling | 116 |

| Discrete frequency domain transform | 118 |

| Identify frequency components in spectrum | 120 |

| Calculate PPG variability measure based on frequency component amplitudes | 122 |

FIG. 4

FIG. 5

FIG. 6

PPG Signal

FIG. 7

FIG. 8A

FIG. 8B

70

72a       74a       76

$PPG_R(t)$ → | AC/DC | (72a)

$PPG_{IR}(t)$ → | AC/DC | (72b)

| $r_{SpO2} \times PPG^n_{IR} - PPG^n_R$ | (74a)

| Digital Filter | (76) → $PPG(t)$ (78)

| $PPG^n_{IR} + c \times PPG^n_R$ | (74b)

**FIG. 9**

| SpO2 algorithm | (82)

70

$PPG_R(t)$

$PPG_{IR}(t)$

| Adaptive Filter | (70)

| Adaptive resampling | (86)

| FFT | (88)

$PPG(f)|_{PR}$
$PPG(f)|_{PR \pm RR}$

| Pleth variability | (90)

| Pulse algorithm | (84)

$PR(t)$

$PPG(f)|_{PR \pm 2RR}$ (92)

PPGV

**FIG. 10**

FIG. 11

FIG. 12

Europäisches
Patentamt

European
Patent Office

Office européen
des brevets

## EUROPEAN SEARCH REPORT

Application Number

EP 22 21 1623

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/287090 A1 (AL-ALI AMMAR [US] ET AL) 6 October 2016 (2016-10-06) * paragraphs [0006], [0007], [0011], [0013], [0015], [0016], [0019], [0058], [0060], [0063], [0064] * ----- | 1-15 | INV. A61B5/024 A61B5/00 |
| A | US 2014/094664 A1 (SOLA I CAROS JOSEP [CH] ET AL) 3 April 2014 (2014-04-03) * paragraphs [0005], [0016], [0038] * ----- | 1-15 | |

TECHNICAL FIELDS
SEARCHED      (IPC)

A61B

The present search report has been drawn up for all claims

| Place of search | Date of completion of the search | Examiner |
|---|---|---|
| The Hague | 17 March 2023 | Papanikolaou, V |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 21 1623

17-03-2023

| Patent document cited in search report | | Publication date | Patent family member(s) | | Publication date |
|---|---|---|---|---|---|
| US 2016287090 | A1 | 06-10-2016 | US | 9307928 B1 | 12-04-2016 |
| | | | US | 2016287090 A1 | 06-10-2016 |
| | | | US | 2019076028 A1 | 14-03-2019 |
| | | | US | 2022400962 A1 | 22-12-2022 |
| US 2014094664 | A1 | 03-04-2014 | EP | 2704623 A1 | 12-03-2014 |
| | | | ES | 2692874 T3 | 05-12-2018 |
| | | | US | 2014094664 A1 | 03-04-2014 |
| | | | WO | 2012150258 A1 | 08-11-2012 |

For more details about this annex : see Official Journal of the European Patent Office, No. 12/82